# EUROPEAN PATENT APPLICATION

(11) **EP 3 804 616 A1**
(43) Date of publication of application: **14.04.2021**
(21) Application number: 20201118.5
(22) Date of filing: 09.10.2020
(51) Int. Cl.: A61B 5/103, A61B 5/107, A43D 1/02, A61B 5/00

(54) **MODULE FOR SCANNING A FOOT, AND ASSOCIATED METHOD OF USE**

(30) Priority: 10.10.2019 US 201962913441 P
(71) Applicant: Techmed 3D Inc., Lévis, Québec G7A 2P7 (CA)
(72) Inventor: DESSERY, Yoann, Lévis, QC G6J 1J4 (CA); LESSARD, Claude, Lévis, QC G6V 7P3 (CA)
(74) Representative: Hoyng Rokh Monegier B.V.

(57) **Abstract**

The scanning module can have a cantilevered foot support extending horizontally and rearwardly from an upward portion, and a base extending horizontally and rearwardly from a bottom of the upward portion, the base overhung by the foot support, the foot support having a transparent portion configured for receiving the bare human feet in a full weight standing position, and a mirror sloping downwardly from a front end of the foot support towards the rear, extending underneath the transparent portion and configured to reflect the image of the plantar face of the foot, as seen across the transparent portion, towards the rear and the sides.

## Description

### FIELD

This specification generally relates to the field of 3D scanning of live subjects with a hand-held scanner.

### BACKGROUND

The process of producing a computer-readable 3D surface model of a physical subject is commonly referred to as 3D scanning. Non-contact, hand-held scanners are available on the market and can be used to obtain a 3D representation of an inanimate object by moving the hand-held scanner around the object, while maintaining the field of view of the scanner oriented toward the object. Non-contact scanning can be done either actively or passively. Active scanning involves emitting a light signal or the like which is reflected on the surface to be scanned, whereas passive scanning does not control emission but typically rather uses stereoscopy, a process which uses two cameras spaced by a known distance and a process associated to triangulation, similar to the process of depth detection known to human binocular vision. Hand-held scanners are typically surface scanners. Surface scanning is different from solid scanning in the sense that in surface scanning only information about the surface of the scanning subject is obtained, to form a shell model of the object, whereas in solid scanning, information about the subject (typically density), is obtained at various depths across it, such as in X-ray computed tomography for instance (CT). While hand-held surface scanning can be performed nowadays using smart phones with associated hardware and software, some specialized devices are also offered on the market, typically offering additional capabilities or features. Any such device which can perform 3D scanning can be considered a hand-held scanner in the context of the following disclosure.

With a hand-held scanner performing surface scanning, for most situations, a scan taken from a single position and orientation will not produce a complete model of the subject, because the surface being scanned hides the opposite face of the subject from the scanner. Accordingly, performing a scan typically involves taking many different depth images (3D images) taken from positions all around the subject, and thus moving the hand-held scanner both in the up-down orientation, and horizontally around the object. The parcels of surface information included in the various depth images need to be brought into a common reference system, a process that will be referred to herein as registration, to create a model inclusive of faces which were visible in only some of the depth images. The depth images typically include at least a depth value for each pixel corresponding to the field of view of the scanner, so the depth images can be 3D images if the scanner has a 2D array of pixels.

Although known hand-held scanner based scanning systems and methods were satisfactory to a certain degree, there remained room for improvement. For instance, in applications pertaining to scanning the feet of persons, which can be required in a medical context, it can be desired for the scan to show the underside of the foot, referred to in the art as the plantar face. It will be understood that the application of a person's full standing weight on the foot changes the shape of the foot, which is especially true for the plantar face. US Patent 9,691,176, the contents of which are hereby incorporated, presents one technique which can be used to facilitate the operation of combining the plantar surfaces scanned on the underside of a foot with the dorsal surfaces scanned on the top of the foot into a 3D model of the foot, but did not allow modelling the foot with the effect of the person's full standing weight. Accordingly, there remained room for improvement.

### SUMMARY

Surface scanning of a foot or feet, can, depending on the intended purpose, require to have a person sit down and apply his/her weight partially on his/her foot/feet, or stand up and apply his/her entire weight on his/her feet. The "entire weight" scenario can allow obtaining a 3D surface model of the foot or feet in a standing condition, and may be required for some uses. Several constraints needed to be taken into consideration in providing a scanning module, especially if it is intended to allow a person to put his/her entire standing weight on the feet while the scan of the entire foot or feet is being performed (i.e. a 3D model of the entire surface of the foot or feet is being acquired). Safety and convenience are definitely amongst these constraints, and for convenience, it can be preferred for the scanning module to be easy to move from one place to another, such as for use and storage for instance. Moreover, especially in the standing scenario, it was preferred for the person not to have to be raised too high up to perform the scan, or for the scanning module to be too big and bulky. Using a transparent platform for the person to stand on, with a sloping mirror underneath, was selected as an avenue having the potential of allowing to position the user at a relatively low height while still allowing to scan the underside of the feet with full standing weight, and was thus considered an interesting avenue, but there remained challenges to be addressed. Indeed, to allow obtaining a full scan of the underside of the feet, it was required to allow scanning from the sides, but it was also desired for the module to be sturdy enough to safely support the weight of a person and therefore required a sufficient structural strength. These latter challenges were addressed by providing a transparent foot platform in a cantilever configuration, with a spacing between the platform and the ground, the spacing bridging the sides and the rear of the module, the foot platform overhanging a sloping mirror which reflected the upward image across the platform horizontally. There remained a challenge associated to distortions in the acquired image. It was found that the distortions in the acquired image can stem from refraction of light as it crosses the transparent platform. This distortion can be alleviated to a level considered acceptable at least in some embodiments, by selecting the sloping angle of the mirror to be around 45 degrees, and by selecting a material having limited refractive index, and having suitable structural characteristics allowing to limit the thickness while reaching weight capacity. Indeed, at 45 degrees mirror inclination, the average path of horizontal light beams can extend at 90 degrees across the transparent platform portion once reflected, an angle of incidence where no refraction, and thus no associated optical distortion, occurs, and the refraction stemming from the non-zero angle field of view of the hand-held scanner can be limited by the limited thickness, and the limited refractive index, of the transparent material.

Accordingly, in accordance with one aspect, there is provided a scanning module for use in performing a computer-readable 3D surface model of a human foot with a hand-held scanner, the scanning module comprising a C-shaped structure including a cantilevered foot support extending horizontally and rearwardly from an upward portion, and a base extending horizontally and rearwardly from a bottom of the upward portion, the base overhung by the foot support, the foot support having a transparent portion configured for receiving the human foot, a mirror sloping downwardly from a front end of the foot support towards the rear, extending underneath the transparent portion and configured to reflect the image of the plantar face of the foot, seen across the transparent portion, towards the rear, and an unobstructed spacing between the foot support and the base bridging the sides and the rear of the module, allowing horizontal visual access to the image of the plantar face of the foot.

In accordance with another aspect, there is provided a method of scanning a foot with a hand-held scanner and a scanning module, the method comprising : standing or sitting a person with his/her full weight or semi-weight on one or both bare feet directly against a raised horizontal transparent support overhanging a sloping mirror of the scanning module; using the hand-held scanner, obtaining a plurality of unobstructed depth images of a dorsal portion of the immobilized foot directly, from corresponding different points of view relative to a ground reference, including moving the hand-held scanner around the immobilized foot while maintaining the hand-held scanner above the plane of the transparent support; using the hand-held scanner, obtaining a plurality of unobstructed depth images of a plantar portion of the immobilized foot indirectly, via the reflection of the mirror and the transparency of the transparent support, from corresponding different points of view relative to a ground reference, including moving the hand held scanner from one side of the scanning module to a rear side of the scanning module while maintaining the hand-held scanner below the plane of the transparent support.

In accordance with another aspect, there is provided a module for imaging a foot, the module comprising a cantilevered foot support extending horizontally and rearwardly from an upward portion, and a base extending horizontally from a bottom end of the upward portion, the base overhung by the foot support, the foot support having a transparent portion, and a mirror sloping downwardly from a front end of the foot support towards the rear, underneath the transparent pane, horizontally reflecting an upward view across the transparent portion, and an unobstructed spacing between the foot support and the base bridging both sides and the rear of the module, across which the horizontal reflection is conveyed both rearwardly and laterally.

Many further features and combinations thereof concerning the present improvements will appear to those skilled in the art following a reading of the instant disclosure.

### DESCRIPTION OF THE FIGURES

In the figures,
Fig. 1 is an oblique view of an example of a scanning module;
Fig. 2 is a side elevation of the scanning module of Fig. 1;
Figs. 3A and 3B schematizes a process of acquiring a 3D surface model of a dorsal portion and of a plantar portion of a foot (Fig. 3A), and assembling these 3D surface models into a whole (Fig. 3B);
Fig. 4 illustrates the horizontal projection of the image of the plantar portion of the foot by the mirror, as viewed across the transparent portion;
Fig. 5A and 5B schematizes lateral displacement of the hand held scanner, and upward-downward displacement of the hand held scanner, respectively;
Fig. 6 schematizes the occurrence of an optical distortion due to refraction across the transparent portion; and
Fig. 7 shows a person applying his/her full standing weight with both feet supported by the transparent portion;
Fig. 8 is an image of a dorsal portion and of a plantar portion of a foot before filtering.

### DETAILED DESCRIPTION

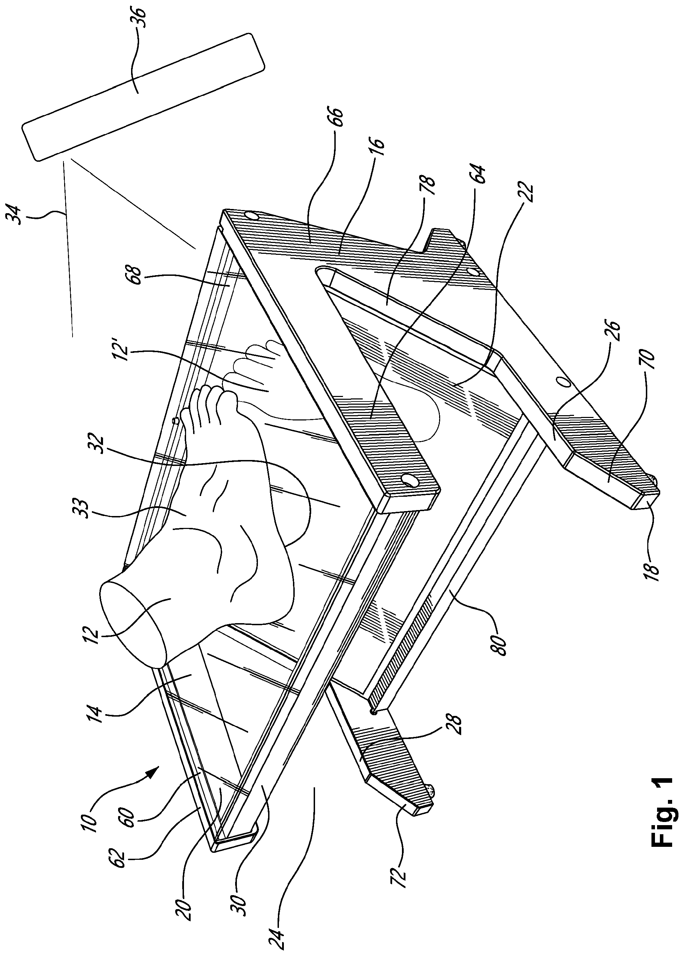

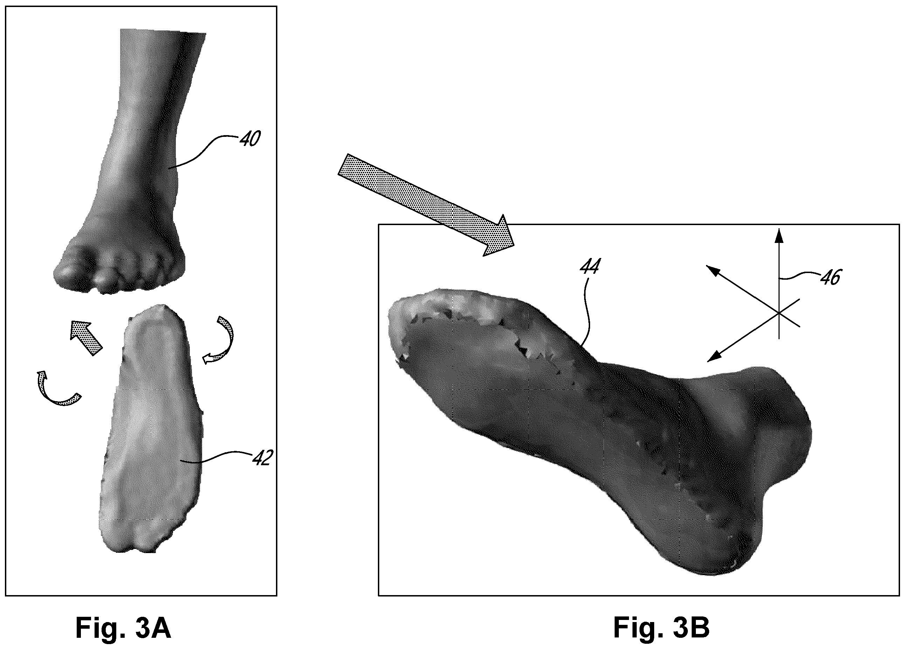

Fig. 1 shows an example of a scanning module 10. The scanning module 10 is adapted for immobilizing a human foot 12 or feet in a manner to allow, using a hand-held scanner 36, to construct a computer-readable 3D surface model (see Fig. 3B) thereof in a full-weight standing position.

The scanning module 10 generally has a C-shaped structure having a foot support 14, an upward portion 16, and a base 18.The foot support 14 is provided in a cantilevered manner and overhangs the base 18. More specifically, the foot support 14 extends horizontally and rearwardly from the upward portion 16, if we arbitrarily determine the upward portion 16 to be at the front for the purpose of relative reference, and the base 18 extends horizontally and rearwardly from a bottom of the upward portion 16, under the foot support 14. At least a portion of the foot support 14 is transparent, and can be referred to as a transparent portion 20. A mirror 22 is also provided. The mirror 22 slopes downwardly from a front end of the foot support 14 towards the rear, and thus extends underneath the transparent portion 20, in a manner to reflect the image of the foot seen across the transparent portion 20 towards the rear and sides. Since the foot support 14 is cantilevered, an unobstructed spacing 24 is provided between the foot support 14 and the base 18 which bridges the sides 26, 28 and the rear 30 of the scanning module 10. The spacing 24 allows visual access to the plantar face 32 of the foot 12 standing on the foot support 14 via the mirror 22 and across the transparent portion 20, and therefore provides access to the field of view 34 of the hand-held scanner 36.

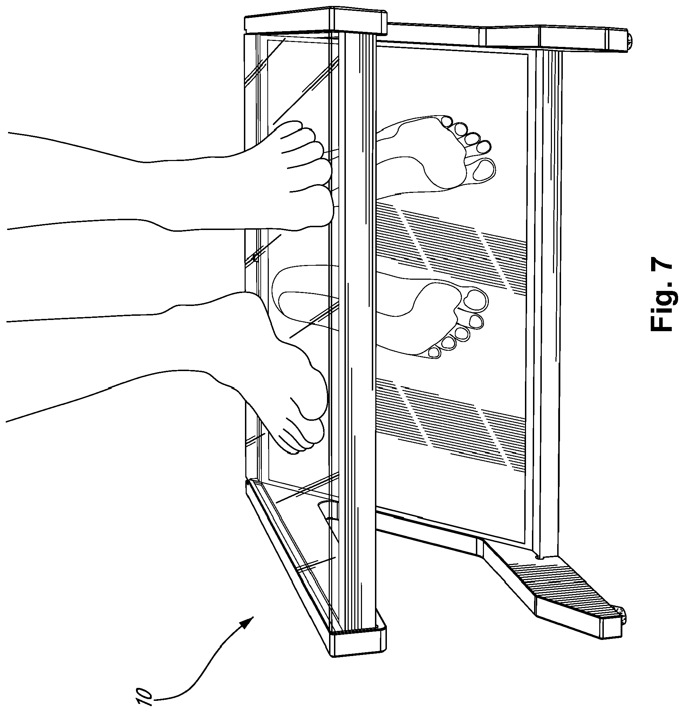

During operation, the hand-held scanner 36 can be configured to repeatedly take 3D images (e.g. a 2D pixel array incorporating depth information at each pixel) as it is moved relatively to the foot 12 or feet (Fig. 7), and the field of view 34 of the hand-held scanner 36 remains oriented towards the foot 12 or feet as it is moved.

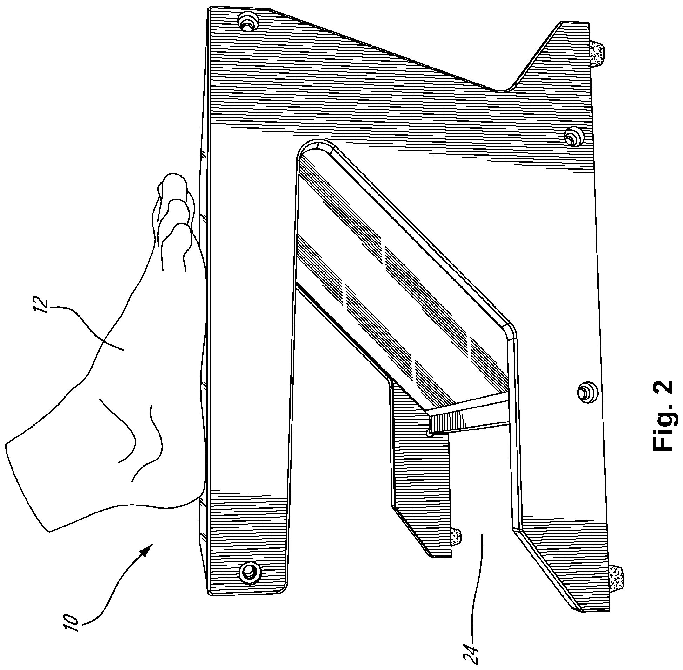

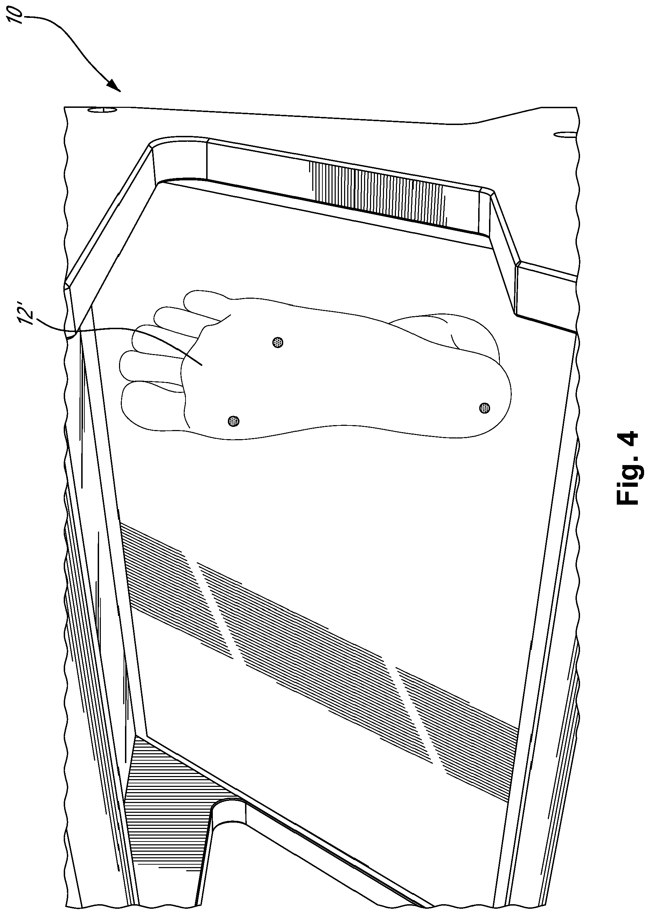

Moving the hand-held scanner 36 around dorsal face/upper portion 33 of the foot 12 such as in the point of view of Fig. 1, down to the sides of the foot such as the point of view of Fig. 2, can lead to a partial 3D surface model 40 of the foot represented in the upper portion of Fig. 3A. This partial 3D surface model of the foot will be referred to as the dorsal face 3D surface model 40, or dorsal face model for short. In the dorsal face model 40, significant portions of the underfoot can be missing, as they are not visible from above the plane of the transparent portion 20 which receives the feet, where the dorsal face model is acquired. The hand-held scanner 36 can then be moved to horizontally target the mirror, in a point of view such as shown in Fig. 4, allowing to view the reflected image of the plantar face. The hand-held scanner 36 can then be moved to the sides, rear, upwards and downwards, while maintaining its field of view 34 aiming the reflected image 12' of the foot 12, through the spacing 24 between the foot support 14 and the base 18, and capture another partial 3D surface model 42 of the foot, this time including surface shape information about the plantar face 32 of the foot 12. This second 3D surface model will be referred to herein as the plantar 3D surface model 42 or plantar model for short, and is also represented in Fig. 3A.

At first glance, it is not easy to determine how the two 3D surface models 40, 42 can be assembled to form a single, complete 3D surface model 44 of the foot 12, as shown in Fig. 3B. Indeed, the dorsal face model 40 is acquired via the foot 12 itself, which is in a first orientation, and is incomplete, missing specifically information about the plantar face, whereas the plantar face model 42 is acquired via a reflected image of the foot 12' which is both rotated and inversed relative to the foot 12, and missing information about the dorsal face. The plantar face model 42, and can further include optical deformations caused by refraction across the transparent portion and/or by imperfections in planarity of the mirror.

However, the inversion of the plantar face model 42 relative to the dorsal face model 40 can be corrected using computer software; imperfections can be minimized by selecting a good quality mirror, orienting the mirror at around 45 degrees (best results being achieved at 45 degrees from the horizontal orientation of the field of view of the hand held scanner), and otherwise as described above; which leaves only the challenge of finding a way to re-orient and re-position the two models 40, 42 into the same frame of reference 46. To assemble the two models to one another, both models 40, 42 can first be acquired and stored individually in a memory accessible to a processor, and via software also provided in a memory accessible to the processor, the two models can be processed if needed, transforming their expression in the memory in case of the latter, and assembled, which can lead to the creation of a third model 44 which can also be stored in the memory.

Two example ways in which this latter challenging process can be achieved will now be detailed. A first way to address this process is to scan the foot and its image in a manner which will allow acquiring a sufficient amount of redundant surface information in both 3D models which will allow to "match" the orientation and position of both models based on this information. This can be achieved on the basis of surface shape data acquired from the foot in the immediate vicinity of the portions of the foot which are in contact with the transparent portion. Indeed, while the portions of the foot which are in contact with the transparent portion will typically be out of reach to the dorsal face scanning process, the rising portions of the foot which are immediately adjacent to the portions which are in contact with the transparent portion may appear in both the dorsal face model and the plantar face model, and can thus be recognized using computer software, and used as a reference to align both models with one another. A second way to address this process is to calibrate the hand-held scanner, or perhaps more specifically the processing software, as a function of the specific geometry of the module, and more specifically of the configuration of the transparent portion and mirror, in a manner for both scanned models to incorporate reference data about the module, in a manner for the reference data about the module to be useable to perform the integration of both 3D models. Indeed, several existing hand-held scanners allow for referencing using reference data about the module, such as position and configuration information about adhesive scanning targets which can be applied to non-transparent and non-reflective surfaces, for instance.

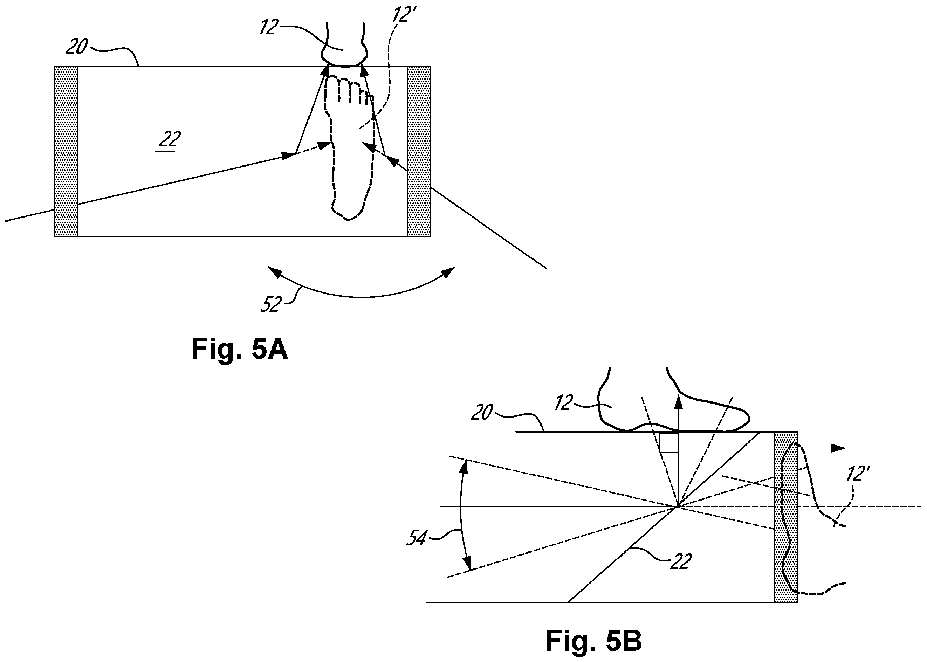

In any event, to avoid the occurrences of "holes" in the 3D surface model 44 of the foot 12, it can be preferred to obtain redundant surface information of the rising surfaces of the foot in the vicinity of the portions of the foot which are in contact with the transparent portion of the foot support. In performing the dorsal face scan, this can be achieved by moving the hand-held scanner 36 in a manner to orient its field of view as close as possible to alignment with the plane of the transparent portion, allowing to "see" as far as possible in the lower region of the foot, and to the extent possible, all around the foot. In performing the plantar face scan, this can be achieved by moving the hand-held scanner from one side to another, can allow the field of view of the hand-held scanner to "see" a bit of the raising portion along corresponding sides of the foot, and moving the hand-held scanner upwardly and downwardly can allow the field of view of the hand-held scanner to "see" a bit of the rising portion on the respective front and back sides of the foot. The "lateral" movement 52 of the hand held scanner 36 from one side of the spacing to the other side of the spacing, which allows to see the sides of the foot, is schematized in Fig. 5A, in which the virtual foot 12' as it appears to the field of view of the hand-held scanner given the reflection by the mirror is shown in dotted lines, whereas the "upward and downward" movement 54 of the hand-held scanner 36 is schematized in Fig. 5B.

It will be understood that errors or aberrations can occur in the 3D model 44 if the 3D model of the plantar face 42 is distorted, or if the redundant surfaces to both the plantar face model 42 and the dorsal face model 44 are otherwise different, and it can be desired to limit such errors or otherwise minimize them. In practice, given that the foot belongs to a human subject, it will not remain perfectly still, and relatively minor movements will lead to correspondingly minor differences and can be tolerated. Another potential source of difference between these regions as they appear in the two 3D models stems from the potential occurrence of refraction of light as it crosses the transparent material which supports the foot. Reasonable attempts can be made to make this transparent material as thin as possible, and in a refractive index as close as possible to the surrounding air medium, but in practice, no perfect material exists which has all the ideal characteristics. As such, the selected material will likely have a non-negligible thickness, and a refractive index which is different from the refractive index of air, while also being transparent and affordable. Poly(methyl methacrylate) (PMMA), for instance, sometimes referred to as acrylic and sold under various trade names, for instance, was found to form a potentially interesting material, but it was still found necessary to provide a significant thickness of PMMA to allow providing a satisfactory maximum weight capacity to suit, say, at least 80 to 90 % of individuals. The target can be to support a maximum weight of 350 lbs, for instance, which may be achieved using a sheet of PMMA having between 16 and 20 mm thick, for instance. PMMA, though offering good structural resistance and transparency, has a refractive index of 1.5, and is thus quite distinct from the refractive index of air which is of 1. Glass may be used in some embodiments, but was not found preferable in the example described herein. Materials other than PMMA or glass may be considered to have suitable transparency and structural resistance characteristics in alternate embodiments.

It was found that the more the sloping angle of the mirror 22 departed from an angle of 45 degrees, the more distortion occurred in the model. It was found that this distortion was caused by the refraction occurring in the transparent portion of the foot support. Indeed, refraction occurs in accordance with Snell's Law, in accordance with the equation **nᵢ∗sine(θᵢ)=nᵣ∗sine(θᵣ)**, where **Θ**ᵢ is the angle of incidence, **Θᵣ** the angle of refraction, **n**ᵢ is the index of refraction of the incident medium, and **n**ᵣ is the index of refraction of the refractive medium.

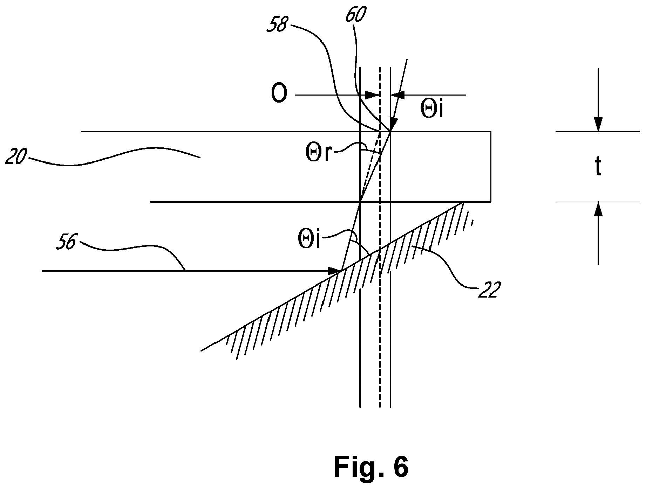

Accordingly, as schematized in Fig. 6 where the thickness t of the transparent portion 20 is exaggerated for the purpose of illustration, a light beam 56 travelling across the transparent medium of the transparent portion 20 will be refracted from the angle **Θ**ᵢ to angle **Θᵣ** upon entry, will travel at angle **Θᵣ** in the transparent medium, and be refracted back to angle **Θ**ᵢ at its exit. Given the thickness t of the foot support's transparent portion 20, the change in travelling angle will create an offset O between the point 58 at which the light beam 56 would have entered the foot support transparent portion would it have travelled in a straight line (i.e would the foot support transparent portion refractive index have been the same as the surrounding medium), and the point 60 at which the light beam truly enters the foot support transparent portion 20 given the difference in refractive index, the thickness t, and Snell's law. Given Snell's Law, this offset O will be greater when the angle of incidence **Θ**ᵢ is greater, and lower when the angle of incidence **Θ**ᵢ is lower, essentially causing a certain level of optical aberration (magnification in certain regions) in the virtual image of the foot as captured in depth images taken by the hand held scanner. The thickness of the glass t will also affect the extent of the offset O in a linear manner.

It will be noted that in active scanning scenarios, not only the image of the foot will be affected by refraction, but the actively sent signal will be affected by refraction as it is reflected by the mirror and transmitted across the transparent portion. Accordingly, the offset O can be doubled in active scanning, leading to an increased amount of distortion in the captured image.

Positioning the mirror 22 at an angle of 45 degrees from the horizontal, and orienting the field of view of the scanner horizontally when scanning the plantar face of the foot, can mitigate these optical aberrations. Indeed, the beams of light which travels horizontally will then hit the transparent foot support at a right angle on average, where no refraction occurs, and the light beams adjacent that horizontal light beam will experience a limited amount of offset O, by contrast with a scenario where the mirror would be positioned at an angle of, say, 30 degrees in otherwise similar conditions. Accordingly, it can be preferred to position the mirror at an angle between 35 and 55 degrees, preferably between 40 and 50 degrees, to limit the effect of refraction on scan quality. It can also be preferred to limit the available field of view of the hand-held scanner, either via hardware adaptations, or via software processes, to narrow the field of view in a manner to favor surface data acquisition at angles relatively close to the horizontal when performing the plantar face scan.

Moreover, it can be preferred to use a transparent material which both a) has structural resistance allowing to safely support the weight of a person, b) be as thin as otherwise possible to minimize the effects of refraction, c) be of a material which has an refractive index as close as feasible to the refractive index of air, and d) has a reasonable cost. In the embodiment illustrated, plexiglass was considered a suitable material to find a balance between these different considerations.

Any suitable hand-held scanner, and associated software, can be used. Some hand-held scanners such as the Go!SCAN™ hand-held scanner manufactured by Creaform for instance, can be designed to recognize a pattern of reflective targets on the scanning module, and facilitate the registration of the 3D images with one another based on the reference provided by the recognized pattern of reflective targets. If using such a hand-held scanner, reflective targets can be positioned on the mirror, or more specifically, a non-reflective non-transparent frame can be positioned along the periphery of the mirror, or along some edges thereof, and this frame can receive scanning targets, for instance, in an effort to calibrate the scanned data with the actual location and configuration of the physical module, and later facilitate registration of the plantar face model with the dorsal face model. However, any alternate suitable hand-held scanner can be used, and some hand-held scanners may not require any external reference other than the comparison made between the 3D surfaces in the different acquired images. In some cases, it can be considered suitable to use a "smart" phone having suitable hardware (e.g. stereoscopic vision with at least two cameras, suitable processor and memory features) and suitable software (e.g. a scanning application) to perform the 3D model of the foot.

In the illustrated embodiment, and perhaps as best seen in Fig. 1, it will be noted that the foot support 10 is comprised of a planar transparent pane 60 extending transversally between cantilevered beams 62, 64. In this case, the transparent pane can be said to constitute a transparent portion of the foot support 20, for instance. Similarly, the C-shaped structure can consist of a plurality of structural members arranged generally in a semi-rectangular-prism shape, with an opening at the rear. More specifically, the upward portion 16 can include two vertical beams 66 which connect the two cantilevered beams 62, 64 of the foot support 20 and an upper transverse beam 68, and the base 18 including two horizontal parallel beams 70, 72 extending rearwardly from corresponding lower ends of the vertical beams 66, at opposite sides. The base 18 can be said to be overhung by the foot support 20. The spacing 24 providing unobstructed visual access is more specifically provided between the rear edge of the foot support 20 and the ground/base beams 70, 72, forming a rear spacing portion, and between rear segments of the cantilevered beams 62, 64 and the base beams 70, 72, forming side spacing portions which are unobstructed over at least a third of the length of the module in the front to rear orientation, preferably about half. Both side spacing portions communicate with the rear spacing portion. In an alternate embodiment, it may be preferred to use a single upward beam between the base and the foot support, or to have a simpler structure.

It will be noted that it can be preferred for the field of view of the mirror to be entirely unobstructed between the planes formed by the base and by the foot support. In practice, a small obstruction can be considered negligible. Moreover, the higher the foot support 20, the greater the angle of observation in the vertical plane. But on the other hand, it may be preferred to limit the height to make the module less bulky, and to make it as safe as possible. Another motivator to limit the height of the module is to make the module more practical for use in a semi-weight scenario, i.e. when a user is sitting on a chair while positioning his/her foot/feet onto the foot support. Making it approximately the height of a standard step was found suitable in some embodiments. In the illustrated embodiment, it was preferred to maintain the height of the foot support within 20-24 cm, and additional angle of observation in the vertical plane was achieved by allowing the mirror 22 to extend all the way down to the ground, between the structural members 72, 70 of the base 18, or more specifically to a vertically thin bracket 80, allowing to gain the equivalent in thickness of the support members 70, 72 by comparison with an embodiment where the mirror would only extend to the top of the support members 70, 72.

Moreover, as shown in Fig. 5A, it can be preferred for the design to allow a relatively wide angle 52 of observation in the horizontal plane, and it will be understood that the angle of observation is limited by the width of the module and by the presence of the mirror, and may also be limited by the presence of structural members on the sides. In this embodiment, it was preferred to clear the side openings as much as possible so as to avoid interference with the angle of observation in the horizontal plane, and therefore avoid obstructing the spacing 24 provided between the foot support 20 and the base 18 and which bridges both sides of the module via the rear. Moreover, on the one hand, it could be preferred to make the scanning module wider, but on the other hand, it can be preferred to avoid making the scanning module too bulky so as to render it inconvenient to move and store when not in use. In the illustrated embodiment, it was preferred to make the scanning module slightly wider than the natural standing position of a relatively large person, in an effort to accommodate 80% or 90% of individuals.

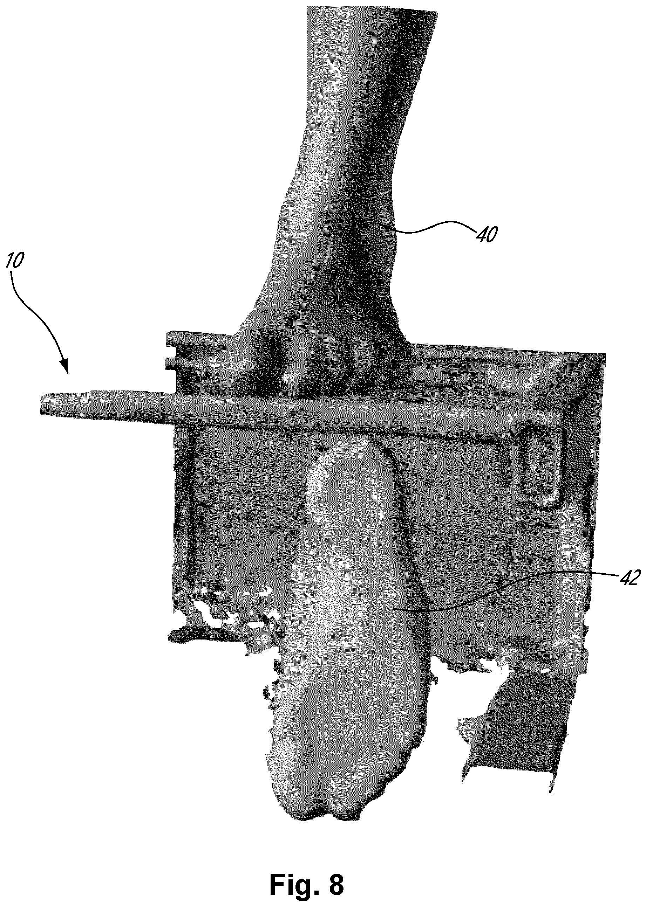

Fig. 8 shows a raw 3D surface model including both the dorsal face of the foot and the plantar face of the foot, but also including a portion of the scanning module. This raw 3D surface model can be treated by filtering out the scanning module, to yield the 3D surface model illustrated in Fig. 3A.

As can be understood, the examples described above and illustrated are intended to be exemplary only. The scope is indicated by the appended claims.

## Claims

1. A scanning module for use in performing a computer-readable 3D surface model of a human foot with a hand-held scanner, the scanning module comprising :
a C-shaped structure including a cantilevered foot support extending horizontally and rearwardly from an upward portion, and a base extending horizontally and rearwardly from a bottom of the upward portion, the base overhung by the foot support, the foot support having a transparent portion configured for receiving the human foot,
a mirror sloping downwardly from a front end of the foot support towards the rear, extending underneath the transparent portion and configured to reflect the image of a plantar face of the foot, as seen across the transparent portion, towards the rear, and
an unobstructed spacing between the foot support and the base, the unobstructed spacing bridging the sides and the rear of the module, allowing horizontal visual access to the image of the plantar face of the foot.

2. The scanning module of claim 1 wherein the foot support includes a planar transparent pane extending transversally between horizontally-extending, cantilevered beams.

3. The scanning module of claim 1 or 2 wherein the upward portion has a frame including two vertical beams each structurally connecting a corresponding one of the cantilevered beams, and a first transversal beam connecting the upper ends of the vertical beams and second transversal beam connecting the lower ends of the vertical beams.

4. The scanning module of any of the previous claims, wherein the base includes two horizontally-extending beams each connecting a lower end of the upward portion, on a respective side, and overhung by the foot support, wherein preferably the mirror extends downwardly between the beams of the base.

5. The scanning module of any of the previous claims, wherein the transparent portion of the foot support is comprised of an acrylic sheet.

6. The scanning module of any of the previous claims, wherein the mirror extends at between 40 and 50 degrees from horizontal.

7. The scanning module of claim 6 wherein the mirror extends at between 42 and 48 degrees from horizontal.

8. The scanning module of claim 6 wherein the mirror extends at 45 degrees from horizontal.

9. The scanning module of any of the previous claims, wherein the foot support extends at 20-24 cm in height relative to a bottom of the base.

10. The scanning module of any of the previous claims, wherein the foot support measures between 35 and 55 cm in transversal width, from one of the sides to the other.

11. A method of scanning a foot with a hand-held scanner and a scanning module, the method comprising :
positioning a person's foot directly against a raised horizontal transparent support, the support overhanging a sloping mirror of the scanning module;
using the hand-held scanner, obtaining a plurality of unobstructed depth images of a dorsal portion of the immobilized foot directly, from corresponding different points of view relative to a ground reference, including moving the hand-held scanner around the immobilized foot while maintaining the hand-held scanner above the plane of the transparent support;
using the hand-held scanner, obtaining a plurality of unobstructed depth images of a plantar portion of the immobilized foot indirectly, via the reflection of the mirror and the transparency of the transparent support, from corresponding different points of view relative to a ground reference, including moving the hand held scanner from one side of the scanning module to a rear side of the scanning module while maintaining the hand-held scanner below the plane of the transparent support.

12. The method of claim 11, wherein the step of obtaining a plurality of unobstructed depth images of a plantar portion of the immobilized foot includes moving the hand held scanner from the rear side of the scanning module to the other side of the scanning module, while orienting the field of view of the hand held scanner towards the mirror, across a spacing provided below a lateral edge of the transparent support.

13. The method of claim 11 or 12, wherein the step of obtaining a plurality of unobstructed depth images of a plantar portion of the immobilized foot includes moving the hand-held scanner upwardly and downwardly, while maintaining the field of view of the hand held scanner oriented towards the mirror, across a spacing provided below a rear edge of the transparent support.

14. The method of any of the claims 11-13, further comprising, using a computer:
Constructing a 3D surface model of the dorsal portion of the immobilized foot using the depth images obtained from the hand held scanner held above the plane of the transparent support;
Constructing a 3D surface model of the plantar portion of the immobilized foot using the depth images obtained from the hand held scanner held below the plane of the transparent support;
recognizing same surface portions redundant to both the dorsal portion 3D surface model and to the plantar portion 3D surface model;
Constructing a 3D surface model of the whole foot by assembling surfaces from the dorsal portion 3D surface model to surfaces from the plantar portion 3D surface model based on the recognized redundant surfaces; and
Storing the 3D surface model of the foot in computer readable memory.

15. The method of claim 14 further comprising displaying the 3D surface model on a computer screen.
